Europäisches Patentamt

European Patent Office

Office européen des brevets

(11) Publication number: **0 445 830 A2**

# EUROPEAN PATENT APPLICATION

(21) Application number: 91103569.9

(22) Date of filing: 08.03.91

(51) Int. Cl.5: **C12P 13/08**, //(C12P13/08, C12R1:19)

The applicant has filed a statement in accordance with Rule 28 (4) EPC (issue of a sample only to an expert). Accession number(s) of the deposit(s): FERM BP-2790, FERM BP-2791 and FERM BP-2792.

(30) Priority: 09.03.90 JP 59669/90

(43) Date of publication of application:
11.09.91 Bulletin 91/37

(84) Designated Contracting States:
DE FR GB

(71) Applicant: KYOWA HAKKO KOGYO CO., LTD.
6-1, Ohte-Machi Itchome
Chiyoda-ku Tokyo(JP)

(72) Inventor: **Kino, Kuniki**
2-2-201, Kyowa-cho
Hofu-shi, Yamaguchi-ken(JP)
Inventor: **Takano, Junichi**
1-2, Kyowa-cho
Hofu-shi, Yamaguchi-ken(JP)
Inventor: **Kuratsu, Yoshiyuki**
2-2-302, Kyowa-cho
Hofu-shi, Yamaguchi-ken(JP)

(74) Representative: **Vossius & Partner**
Siebertstrasse 4 P.O. Box 86 07 67
W-8000 München 86(DE)

(54) Process for producing L-threonine.

(57) Disclosed is a process for producing L-threonine which comprises culturing in a medium a microorganism belonging to the genus Escherichia and having resistance to at least one of L-serine and ethionine and an ability to produce L-threonine until L-threonine is accumulated in the culture, and recovering L-threonine therefrom.

EP 0 445 830 A2

The present invention relates to a process for producing L-threonine by culturing a microorganism belonging to the genus Escherichia and having resistance to at least one of L-serine and ethionine and an ability to produce L-threonine. L-Threonine is an amino acid which is not only useful as a medicament such as an amino acid preparations but also utilizable as an additive for animal feed.

Heretofore, various processes for producing L-threonine by culturing microorganisms belonging to the genus Escherichia are known; for example a process using a microorganism having a sensitivity to borrelidin (Japanese Published Examined Patent Application No. 6752/76); a process using a microorganism which requires diaminopimelic acid and methionine for growth and of which threonine biosynthesis system is resistant to the feedback inhibition of threonine (Japanese Published Examined Patent Application No. 10037/81); a process using a microorganism having resistance to at least one of rifampicin, lysine, methionine, aspartic acid and homoserine, or a decreased ability to degrade L-threonine (Japanese Published Unexamined Patent Application No. 273487/88, EP-A-237819); etc. Furthermore, as processes using microorganisms other than those belonging to the genus Escherichia, there are known a process using a microorganism belonging to the genus Serratia which is deficient in threonine dehydrogenase and resistant to threonine metabolic antagonist (Japanese Published Examined Patent Application No. 48195/77); a process using a microorganism belonging to the genus Corynebacterium, having resistance to α-amino-β-hydroxyvaleric acid and S-(2-aminoethyl)-L-cysteine, and a requirement for methionine (Japanese Published Unexamined Patent Application No. 19087/72); a process using a microorganism belonging to the genus Brevibacterium, having resistance to α-amino-β-hydroxyvaleric acid and S-(2-aminoethyl)-L-cysteine and a requirement for L-leucine (Japanese Published Unexamined Patent Application No. 31093/75); a process using a microorganism belonging to the genus Brevibacterium, having resistance to α-amino-β-hydroxyvaleric acid and S-(2-aminoethyl)-L-cysteine, and a requirement for L-isoleucine and L-lysine (Japanese Published Unexamined Patent Application No. 224684/83); a process using a mutant belonging to the genus Serratia and having resistance to a methionine metabolic antagonist (Japanese Published Unexamined Patent Application No. 134993/81, U.S. Patent No. 4463094); a process using a mutant belonging to the genus Providencia and having resistance to a methionine metabolic antagonist (Japanese Published Examined Patent Application No. 18478/88); etc.

According to the present invention, L-threonine can be produced in high yields at low costs by culturing in a medium a microorganism belonging to the genus Escherichia and having resistance to at least one of L-serine and ethionine and an ability to produce L-threonine until L-threonine is accumulated in the culture, and recovering L-threonine therefrom.

As the microorganism used in the present invention, any microorganism may be used so long as it belongs to the genus Escherichia and has resistance to at least one of L-serine and ethionine and an ability to produce L-threonine.

The suitable mutant strains to be used in the present invention may be obtained by subjecting a microorganism belonging to the genus Escherichia and having an ability to produce L-threonine to a conventional mutation technique in order to endow at least one of L-serine and ethionine resistance with the microorganism. Alternatively, the mutant strains may also be obtained by a reverse process, i.e. by imparting L-threonine productivity such as endowment of nutrient requirement or threonine metabolic antagonist-resistance, etc. to a mutant strain having resistance to at least one of L-serine and ethionine derived from a wild strain. Preferred examples are Escherichia coli H-7701, H-7702 and H-7729. The L-threonine-producing strains belonging to the genus Escherichia are advantageous in that amino acids of residual products are minimized.

Production of L-threonine can be effected by culturing the above microorganisms in a conventional manner. As the medium used, any synthetic or natural medium may be used so long as it appropriately contains carbon sources, nitrogen sources, inorganic materials, and other trace amounts of nutrients required for the strain used.

As the carbon sources, carbohydrates such as glucose, fructose, lactose, molasses, cellulose hydrolyzate, hydrolyzate of crude sugar, starch hydrolyzate, etc.; and organic acids such as pyruvic acid, acetic acid, propionic acid, fumaric acid, malic acid, formic acid, lactic acid, etc. can be used. Depending upon assimilability of a microorganism to be used, glycerine, alcohols such as ethanol, etc. may also be used.

As the nitrogen sources, ammonia; ammonium salts of inorganic or organic acids such as ammonium chloride, ammonium sulfate, ammonium acetate, ammonium phosphate, etc.; amines; other nitrogen-containing compounds; peptone, meat extract, corn steep liquor, casein hydrolyzate, soybean cake hydrolyzate, various cultured cells and their digested product, etc. can be used.

As the inorganic compounds, potassium dihydrogen phosphate, dipotassium hydrogen phosphate, magnesium phosphate, magnesium sulfate, sodium chloride, ferrous sulfate, manganese sulfate, copper sulfate, calcium carbonate, etc. can be used.

Culturing is carried out under aerobic conditions such as deep shaking culture, submerged culture with aeration, etc. The temperature for the culturing is in a range of 20 to 40°C, preferably in a range of 25 to 38°C. The pH of the medium is maintained at 5 to 9, preferably around neutrality during the culturing. Control of pH in the medium is carried out with calcium carbonate, an inorganic or organic acid, an alkaline solution, ammonia, a pH buffer, etc. Usually, after culturing for 2 to 7 days, L-threonine is accumulated in the culture.

After the completion of culturing, precipitates such as cells, etc. are removed from the culture. By using ion exchange treatment, concentration, salting out, etc. in combination, L-threonine can be recovered from the resulting culture.

Hereafter the present invention is specifically described with reference to the examples.

Example 1 Acquirement of the mutant strain of the present invention

Escherichia coli H-7700 having no requirement for diaminopimelic acid was derived from Escherichia coli H-4581 (FERM BP-1411) having diaminopimelic acid requirement, methionine requirement, α-amino-β-hydroxyvaleric acid resistance, reduced L-threonine decomposition ability, rifampicin resistance, lysine resistance, methionine resistance, homoserine resistance and aspartic acid resistance. Escherichia coli H-7700 was subjected to a conventional mutation treatment with N-methyl-N'-nitro-N-nitrosoguanidine (0.2 mg/ml, 30°C, 30 minutes). The treated cells were smeared on a minimum medium (0.5% glucose, 0.2% $NH_4Cl$, 0.2% $KH_2PO_4$, 0.01% $MgSO_4 \cdot 7H_2O$, 20 mg/l $FeSO_4 \cdot 7H_2O$, 50 mg/l DL-methionine and 2% agar; pH 7.2) containing DL-ethionine (10 g/l). After the incubation at 30°C for 2 to 6 days, large colonies were grown as the ethionine-resistant strain. The strains were picked up and subjected to the L-threonine production test. A mutant having a higher L-threonine productivity than that of H-7700 strain was selected. The selected strain has been named Escherichia coli H-7702. The strain has been deposited under the Budapest Treaty with the Fermentation Research Institute (FRI), the Agency of Industrial Science and Technology of Japan since March 6, 1990 under the access number FERM BP-2791. The parent strain H-7700 did not grow in the aforesaid minimum medium containing DL-ethionine (cf. Table 1).

Each of Escherichia coli H-7700 and Escherichia coli H-7702, was subjected to a mutation treatment with N-methyl-N'-nitro-N-nitrosoguanidine (0.2 mg/ml, 30°C, 30 minutes), and the treated cells were smeared on a minimum medium (0.5% glucose, 0.2% $NH_4Cl$, 0.2% $KH_2PO_4$, 0.01% $MgSO_4 \cdot 7H_2O$, 20 mg/l $FeSO_4 \cdot 7H_2O$, 50 mg/l DL-methionine and 2% agar; pH 7.2) containing L-serine (7 g/l). After the incubation at 30°C for 2 to 6 days, large colonies were grown as the L-serine-resistant strain. The strains were picked up and subjected to the L-threonine production test. A mutant derived from H-7700 and having a higher L-threonine productivity than that of H-7700 was selected. The selected strain has been named Escherichia coli H-7701. A mutant derived from H-7702 and having a higher L-threonine productivity than that of H-7702 was selected. The selected strain has been named Escherichia coli H-7729. The strains H-7701 and H-7729 have been deposited under the Budapest Treaty with the FRI since March 6, 1990 under access numbers FERM BP-2790 and FERM BP-2792, respectively. The parent strain Escherichia coli H-7700 or H-7702 did not grow in the aforesaid minimum medium containing L-serine (cf. Table 1)

Table 1 shows the growth of the above-mentioned strains when cultured at 30°C for 3 days on a minimal agar plate medium containing DL-ethonine or L-serine in different concentrations.

## Table 1

| Concentration of DL-Ethionine (g/l) | | | |
|---|---|---|---|
| Strain | 0 | 3 | 10 |
| H-7700 | + | ± | - |
| H-7702 | + | + | + |

| Concentration of L-Serine (g/l) | | | |
|---|---|---|---|
| Strain | 0 | 3 | 7 |
| H-7700 | + | ± | - |
| H-7701 | + | + | + |
| H-7702 | + | ± | - |
| H-7729 | + | + | + |

+: sufficient growth

±: moderate growth

-: no growth

Example 2 Test on L-Threonine Productivity

Using the mutant strains obtained in Example 1, tests on productivity of L-threonine by fermentation were carried out. Escherichia coli H-7700, Escherichia coli H-7701, Escherichia coli H-7702 or Escherichia coli H-7729 was cultured with shaking at 30°C for 16 hours, in a seed medium (pH 7.4) comprising 2% glucose, 1% peptone, 1% yeast extract and 0.25% NaCl. 100 mℓ of the resulting seed culture was inoculated into 1 liter of a fermentation medium having the following composition charged in a 2ℓ-small fermentation tank and culturing was carried out at 30°C at 800 rpm and an aeration rate of 1 liter/min for 80 hours. During the culturing, pH control and supply of nitrogen source were made by aqueous ammonia, whereby the pH was kept at about 6.5±0.2, and glucose is supplied where appropriate. After the completion of the culturing, the amount of L-threonine accumulated was quantitatively determined by high performance liquid chromatography. The results are shown in Table 2. Composition of the fermentation medium:

4% glucose, 1.2% $(NH_4)_2SO_4$, 0.2% $KH_2PO_4$, 0.01% $MgSO_4 \cdot 7H_2O$, 0.5% corn steep liquor and 0.3 g/l DL- methionine, pH 7.4

4

Table 2

| Strain | L-Threonine (g/l) |
|--------|-------------------|
| H-7700 | 3.1 |
| H-7701 | 12.2 |
| H-7702 | 18.3 |
| H-7729 | 38.4 |

One liter each of the L-threonine-containing fermentation broth obtained by culturing each of H-7702 strain and H-7729 strain was centrifuged (3000 rpm, 10 minutes) to remove the cells and other impurities. The obtained supernatant was passed through a column packed with strongly acidic cation exchange resin Diaion SKIB (K type) to adsorb L-threonine thereto. After the column was washed with water, the column was eluted with 0.5N aqueous ammonia to collect L-threonine fractions. The collected fractions were concentrated and ethanol was added. By storing the mixture under cooling, 15.0 g in case of Escherichia coli H-7702, and 30.6 g in case of Escherichia coli H-7729, of L-threonine crystals were obtained in a purity of 98% or higher.

**Claims**

1. A process for producing L-threonine which comprises culturing in a medium a microorganism belonging to the genus Escherichia and having resistance to at least one of L-serine and ethionine and an ability to produce L-threonine until L-threonine is accumulated in the culture, and recovering L-threonine therefrom.

2. The process according to claim 1, wherein said microorganism belongs to the species Escherichia coli.

3. The process according to claim 2, wherein said microorganism is Escherichia coli H-7701 (FERM BP-2790) having resistance to L-serine, Escherichia coli H-7702 (FERM BP-2791) having resistance to ethionine or Escherichia coli H-7729 (FERM BP-2792) having resistance to L-serine and ethionine.

4. A biologically pure culture of Escherichia coli H-7701 (FERM BP-2790).

5. A biologically pure culture of Escherichia coli H-7702 (FERM BP-2791).

6. A biologically pure culture of Escherichia coli H-7729 (FERM BP-2792).